(19) **Europäisches Patentamt / European Patent Office / Office européen des brevets**

(11) **EP 4 199 952 B1**

(12) **EUROPEAN PATENT SPECIFICATION**

(45) Date of publication and mention of the grant of the patent:
**28.01.2026 Bulletin 2026/05**

(21) Application number: **21759162.7**

(22) Date of filing: **19.08.2021**

(51) International Patent Classification (IPC):
*A61K 38/18* (2006.01)     *A61K 8/64* (2006.01)
*A61P 17/00* (2006.01)     *A61K 9/00* (2006.01)
*A61Q 19/04* (2006.01)     *A61K 45/06* (2006.01)

(52) Cooperative Patent Classification (CPC):
**A61P 17/00; A61K 8/64; A61K 9/0014; A61K 9/0017; A61K 38/185; A61K 45/06; A61Q 19/04**

(86) International application number:
**PCT/IB2021/057631**

(87) International publication number:
**WO 2022/038555 (24.02.2022 Gazette 2022/08)**

(54) **PEPTIDE FOR THERAPEUTIC APPLICATIONS IN THE DERMATOLOGICAL FIELD**

PEPTID FÜR THERAPEUTISCHE ANWENDUNGEN AUF DERMATOLOGISCHER BASIS

PEPTIDE POUR APPLICATIONS THÉRAPEUTIQUES DANS LE DOMAINE DERMATOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2020 IT 202000020287**

(43) Date of publication of application:
**28.06.2023 Bulletin 2023/26**

(73) Proprietor: **Dompé farmaceutici S.p.A.**
**20122 Milan (IT)**

(72) Inventor: **PELLEGRINI, Pablo**
**20123 Milano (IT)**

(74) Representative: **Dompé farmaceutici Spa**
**Via San Martino, 12-12/a**
**20122 Milano (IT)**

(56) References cited:
**WO-A1-2011/049758     WO-A1-2020/075108**
**WO-A2-2013/065078     CN-A- 1 616 086**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention relates to a peptide capable of promoting skin pigmentation and/or innervation in skin dyschromia and/or de-innervation diseases. A pharmaceutical composition comprising such peptide and at least one pharmaceutically acceptable excipient are a further object of the invention.

**PRIOR ART**

Nerve growth factor (NGF) is a neurotrophic and neuropeptide factor primarily involved in regulating the growth, maintenance, proliferation and survival of certain target neurons.

NGF was discovered by Prof. Rita Levi-Montalcini, at the Zoology Institute of the Washington University of St. Louis (Levi-Montalcini R., Harvey Lect., 60:217, 1966), and her discovery represented a notable progress in the study of the mechanisms of growth and differentiation of the nerve cell, as NGF is able to influence the development and conservation of the biological functions of neurons and their regeneration.

Numerous in vitro and in vivo studies have demonstrated the pathophysiological importance of NGF in the prevention of neuronal damage of a surgical, chemical, mechanical and ischemic nature, making it the ideal candidate for the treatment of various conditions of the central and peripheral nervous system (Hefti F. , J. Neurobiol., 25: 1418, 1994; Fricker J., Lancet, 349:480, 1997). In fact, for many years clinical studies have been carried out on patients suffering from Parkinson's disease and Alzheimer's disease, by intracerebral administration of murine NGF (see, for example, Olson L. et al., J. Neural Transm, (Parkinson's Disease and Dementia Section), 4: 79-95, 1992). The results of these studies confirmed the observations made in animal models and showed the absence of possible side effects following the administration of murine NGF. This feature was later confirmed for human recombinant NGF (Petty BG et al., Annals of Neurology, 36:244-246, 1994).

Immune, neuronal and skin cells play a key role in the onset of various dermatological pathologies associated with dyschromia, such as psoriasis, atopic dermatitis, vitiligo, herpes.

NGF receptors are expressed on sensory nerves, keratinocytes, melanocytes, fibroblasts, hair follicles and various immune cells, playing an important role in skin homeostasis.

Keratinocytes are a key cellular component of both homeostasis and pathophysiological processes in the skin and have been shown to produce a number of cytokines and to be stimulated by various growth factors. NGF mRNA has been shown in vitro to be maximal when keratinocytes are in exponential growth phase (Growth-regulated synthesis and secretion of biologically active nerve growth factor by human keratinocytes.J Biol Chem 1991; 266: 21718-21722).

In keratinocytes of various species, NGF regulates proliferation and differentiation, and protects those of human nature from apoptosis by activating the high affinity receptor (trkA), showing a known anti-aging and skin regeneration activity (Nerve growth factor: its significance in cutaneous biology. J Invest Dermatol Symp Proc 2: 31-36, 1997), and for maintaining the integrity of the epidermis.

Indeed, Trka plays a key role in activating the signal transduction pathways that promote growth and survival, and which are also involved in skin innervation processes.

In melanocytes, survival and dendrite formation is increased by NGF after UV irradiation (The trk family of receptors mediates nerve growth factor and neurotrophin-3 effects in melanocytes J. Clin. Invest. 94: 1550-1562, 1994).

The prior art shows how the regulation of NGF can favor the treatment of both skin diseases associated with dyschromic phenomena, and diseases associated with de-innervation, such as viral rashes, such as herpes, and rare syndromes such as trigeminal trophic, which often occurs in areas previously affected by herpes zooster.

The treatment of vitiligo is currently entrusted to therapies that have significant side effects (Am. J. Clin. Dermatol., 2017, 18:733-744). In particular, treatment with corticosteroids, administered both topically and orally, cannot be prolonged beyond 3 weeks. Even the treatment of choice represented by narrow-band phototherapy (NB-UVB phototherapy), despite the average quality of the results obtained (repigmentation greater than 75% in 63% of cases) can cause acute undesirable effects such as itching, erythema, burns, xerosis.

The international publication WO2013065078A2 revealed that the topical administration on the skin of preparations containing NGF is effective in obtaining an intensification of the skin color, i.e. an increase in pigmentation, in the case of healthy skin, not affected by dyschromatosis, as well as an improvement in the dermatological conditions involving skin achromia or hypochromia, as in the case of vitiligo.

WO 2020/075108 A1 discloses the cosmetic use of NGF1-14 for the treatment of healthy skin to reduce skin aging and photo-aging.

Despite research developments, there is still a problem of improving the therapy of skin discolorations and skin de-innervation diseases.

## SUMMARY OF THE INVENTION

The references to methods of treatment in the subsequent paragraphs of this description are to be interpreted as references to the compounds, pharmaceutical compositions and medicaments of the present invention for use in a method for therapeutic treatment of the human (or animal) body by therapy.

The Applicant has faced the problem of improving the therapy of skin dyschromias and skin de-innervation diseases. In particular, the Applicant has focused its research in the field of peptides derived from NGF, finding that the NGF 1-14 peptide having the sequence ID No. 1 is particularly effective in activating the production of melanin by melanocytes.

As will be demonstrated in Example 1 of the experimental part, the NGF 1-14 peptide tested in vitro on primary cell cultures of normal human melanocytes results in a comparable increase in the melanin content per cell compared to the whole NGF protein, albeit at higher concentrations (2-3 log).

Surprisingly with respect to the activity demonstrated in vitro, as will be demonstrated in Example 2a, the NGF 1-14 peptide tested in vivo on a mouse model of human vitiligo shows a more uniform and homogeneous repigmentation than the whole protein, at the same dose; as shown in Example 2b, in vivo the NGF 1-14 peptide surprisingly determines a greater increase in the density of the nerve endings, at the same dose.

As will be shown in Example 3, the NGF 1-14 peptide administered by intraplantar route to Sprague-Dawley rats shows much higher tolerability even at the maximum dose, compared to NGF. The result of this test is very important in light of the fact that the pain caused at the site of administration by NGF and consequently the poor skin tolerability of the whole protein administered intradermally or intramuscularly is known. This effect has been extensively studied and has been shown to be intrinsically linked to the mechanism of action, in particular to the activation of NGF receptors. For this reason, the observed tolerability of the NGF 1-14 peptide administered intradermally is surprising.

A further advantage of the NGF1-14 peptide with respect to the whole protein is represented by the fact that it can be easily synthesized with conventional peptide synthesis approaches and stored under mild conditions and for prolonged periods, with a consequent significant reduction in production costs.

The Applicant also noted that some changes in the number and type of amino acids of the NGF1-14 peptide can be made without substantially altering the activity thereof.

Therefore, a first object of the present invention relates to a peptide of 14 amino acids having the sequence ID No. 1 or a peptide of up to 16 amino acids having a sequence with at least 85%, preferably at least 90%, and preferably at least 95% identity with sequence ID No. 1 and/or salt thereof, for use in the therapeutic treatment and/or prevention of skin dyschromia and/or de-innervation diseases of the skin.

A second object of the present invention relates to a pharmaceutical composition consisting of a peptide of 14 amino acids having the sequence ID No. 1 or a peptide of up to 16 amino acids having a sequence with at least 85%, preferably at least 90%, and preferably at least 95% identity with sequence ID No. 1 and/or salt thereof, and at least one pharmaceutically acceptable excipient, for use in the therapeutic treatment and/or prevention of skin dyschromia and/or de-innervation diseases of the skin.

## BRIEF DESCRIPTION OF THE FIGURES

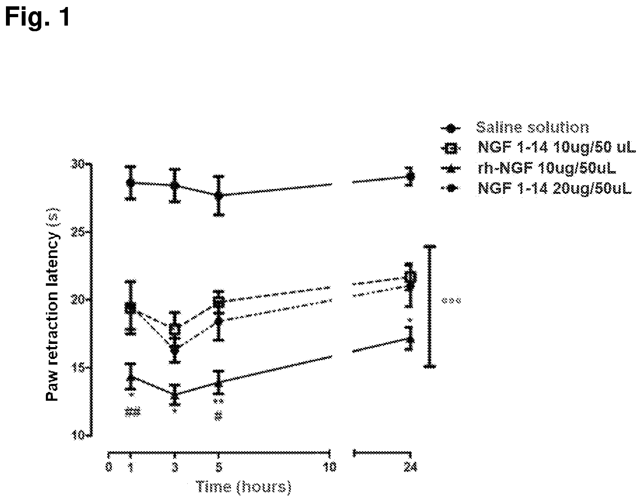

Figure 1 shows the paw retraction latency of animals treated with saline (black circle, solid line), with NGF 1-14 10 $\mu$g/50 $\mu$L (white square), NGF 1-14 20 $\mu$g/50$\mu$L (black circle - broken line) and rh-NGF 10 $\mu$g/50$\mu$L mL (black triangle) at 1, 3, 5 and 24 hours after injection.

## DETAILED DESCRIPTION OF THE INVENTION

Preferably, in an embodiment according to the first object of the present invention, the peptide has a length of 14 amino acids and sequence ID No. 1 and/or salt thereof.

The sequence ID No. 1 according to the present invention is represented by the sequence SSSHPIFHRGEFSV.

The abbreviations of the amino acid sequences used herein comply with the IUPAC-IUB nomenclature shown in the following Table A.

Table A

| Alanine | Ala | A | Arginine | Arg | R |
|---|---|---|---|---|---|
| Asparagine | Asn | N | Aspartic acid | Asp | D |
| Cysteine | Cys | C | Glutamic acid | Glu | E |
| Glutamine | Gln | Q | Glycine | Gly | G |
| Histidine | His | H | Isoleucine | Ile | I |

(continued)

| Leucine | Leu | L | Lysine | Lys | K |
| Methionine | Met | M | Phenylalanine | Phe | F |
| Proline | Pro | P | Serine | Ser | S |
| Threonine | Thr | T | Tryptophan | Trp | W |
| Tyrosine | Tyr | Y | Valine | Val | V |

The peptides according to the invention can have at least 85%, at least 90% and at least 95% identity with the sequence ID No. 1 when they are optimally aligned. Optimal sequence alignment can be conducted with various known methods and with the computer implementation of known algorithms (e.g. BLAST, TFASTA, BESTFIT, as in Wisconsin's Genetics Software Package, Release 7.0, Genetics Computer Group, Madison, WI). The BLAST algorithm can also be used (Altschul et al., Mol. Biol. (1990), 215, 403-410) whose software can be obtained from the National Center for Biotechnology Information www.ncbi.nlm.nih.gov/.

By "percentage sequence identity" with respect to a peptide sequence it is meant the percentage of identical residues in two sequences. The percentage sequence identity (% SI) is calculated with the following formula:

$$\%SI = (nt-nd) \times 100/nt$$

where nt is the number of residues in the base sequence and nd is the total number of non-identical residues in the compared sequence when aligned so that the maximum number of amino acids is identical. Consequently, an SSSHPIFHRG*D*FDFSV sequence will have a sequence identity of approximately 92.8% with sequence ID No. 1 (nd=1 and nt=14).

Preferably, in another embodiment according to the first object of the present invention, the peptide has a length of up to 16 amino acids and a sequence with at least 85%, preferably at least 90%, and preferably at least 95% identity with the sequence ID No. 1, and/or salt thereof; more preferably, according to such embodiment, said peptide has a length of up to 15 amino acids, more preferably of 14 amino acids.

The variation of the amino acid sequence in the peptides comprising the ID No. 1 of the present invention comprises the conservative substitution of amino acids which do not affect peptide activity. Substitutions capable of maintaining peptide activity are selected based on (a) effectiveness in maintaining the backbone structure of the peptide in the substitution area, such as three-dimensional foil or helical structures, (b) effectiveness in maintaining the electric charge or the hydrophobicity of the molecule in the target area, or (c) effectiveness in maintaining the bulk of the side chain.

Examples of conservative substitution belong to the group of basic amino acids (arginine, lysine and histidine), acid amino acids (glutamic and aspartic acids), polar amino acids (glutamine and asparagine), hydrophobic amino acids (leucine, isoleucine, valine and methionine), aromatic amino acids (phenylalanine, tryptophan and tyrosine) and small amino acids (glycine, alanine, serine and threonine).

Substitutions of amino acids which generally do not alter the specific activity are known in the art of the present invention.

The most common alterations are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, and the opposite alterations. Another example of conservative substitution is shown in the following Table C.

TABLE C

| Starting amino acid | Possible substitution | Preferred substitution |
|---|---|---|
| Ala (A) | V; L; I | V |
| Arg (R) | K; Q; N | K |
| Asn (N) | Q; H; D, K; R | Q |
| Asp (D) | E; N | E |
| Cys (C) | S; A | S |
| Gln (Q) | N; E | N |
| Glu (E) | D; Q | D |
| Gly (G) | A | A |
| His (H) | N; Q; K; R | R |

(continued)

| Starting amino acid | Possible substitution | Preferred substitution |
|---|---|---|
| Ile (I) | L; V; M; A; F; | L |
| Leu (L) | I; V; M; A; F | I |
| Lys (K) | R; Q; N | R |
| Met (M) | L; F; I | L |
| Phe (F) | L; V; I; A; Y | Y |
| Pro (P) | A | A |
| Ser (S) | T | T |
| Thr (Thr) | S | S |
| Trp (W) | Y; F | Y |
| Tyr (Y) | Trp; F; T; S | F |
| Val (V) | I; L; M; F; A; | L |

Depending on its length, the peptide of the present invention can be synthesized by a method well known in the art, for example, by an automatic peptide synthesizer, or produced by a genetic engineering technology. For example, a fusion gene encoding a fusion protein which includes a fusion partner and the peptide of the present invention is prepared by genetic engineering and then transformed into a host cell to express the fusion protein. Next, the peptide of the present invention is cleaved and isolated from the fusion protein using a protease or a compound so as to produce the desired peptide. To this end, between the polynucleotides encoding the fusion partner and the peptide of the present invention, a DNA sequence can be inserted which encodes the amino acid residues which can be cleaved by a protease such as the factor Xa or enterokinase, or a compound such as CNBr or hydroxylamine.

Preferably, in an embodiment according to the second object of the present invention, the pharmaceutical composition comprises from 10 to 10,000 μg/mL, preferably from 100 to 5,000 μg/mL, and more preferably from 200 to 4,000 μg/mL of a peptide having a length of 14 amino acids and sequence ID No. 1 and/or salt thereof.

Preferably, in another embodiment according to the second object of the present invention, the pharmaceutical composition comprises from 10 to 10,000 μg/mL, preferably from 100 to 5,000 μg/mL, and more preferably from 200 to 4,000 μg/mL of a peptide having a length of up to 16 amino acids and a sequence with at least 85%, preferably at least 90%, and preferably at least 95% identity with sequence ID No. 1 and/or salt thereof.

According to the second object of the present invention, the pharmaceutical composition can contain a variety of other optional components suitable for making such compositions more pharmaceutically acceptable or for providing them with further benefits of use. Such conventional optional ingredients are well known to those skilled in the art. These include all pharmaceutically acceptable ingredients such as those described for example in Rowe et al.; "Handbook of Pharmaceutical Excipients", 2009, Pharmaceutical Press.

The type of vehicle or excipient used in the present invention depends on the type of product desired. The compositions useful in the present invention can be a large variety of product forms. These include, but are not limited to, lotions, creams, gels, sticks, sprays, ointments, pastes, mousses.

These product forms may include several types of vehicles, including, but not limited to, solutions, aerosols, emulsions (including oil-in-water and water-in-oil), gels, solid compositions and liposomes.

The compositions of the present invention can comprise water, in different amounts depending on the form of the composition. The amount of water, if present, can range from less than 1% to more than 99% by weight with respect to the weight of the total composition. The aqueous compositions of the present invention are particularly formulated as aqueous lotions or as aqueous emulsions or as water-in-oil or oil-in-water emulsions or as multiple emulsions (triple oil-in-water-in-oil or water-in -oil-in-water emulsion).

The solid compositions, spray compositions and water-in-oil creams generally consist of quantities of water lower than 10%, preferably lower than 5% by the total weight of the composition. The roll-on compositions, aqueous compositions and deodorants generally consist of an amount of water ranging from about 15% to about 99%, preferably from about 30% to about 90%, even more preferably from about 50% to about 80%, by weight with respect to the total weight of the composition.

The compositions of the present invention can comprise one or more volatile solvents. If present, the volatile solvent or solvent mixture will generally be at a level between about 10% and about 90%, preferably between about 25% and about 75%, even more preferably between about 35% and about 65%, by weight with respect to the total weight of the composition. The solvents used herein are preferably volatile organic solvents.

As used herein, the term "volatile" refers to substances with a significant amount of vapor pressure under ambient conditions, as understood by those skilled in the art.

The volatile solvents to be used herein will preferably have a vapor pressure of about 2kPa or more, preferably about 6kPa or more, at 25 °C. The volatile solvents to be used will preferably have a boiling point in a normal atmosphere (1 atm) lower than about 150 °C, more preferably lower than about 100 °C, even more preferably lower than about 90 °C, and even more preferably lower than about 80 °C.

Preferably, the volatile solvents to be used herein will be relatively odorless and safe for use on human skin. Suitable volatile solvents include, but are not limited to $C_1$-$C_4$ alcohols, volatile silicones and mixtures thereof. The preferred volatile solvents are $C_1$-$C_4$ alcohols and mixtures thereof. Ethanol is the preferred solvent for the present use.

The compositions of the present invention can also comprise one or more non-volatile solvents. If present, the non-volatile solvent or solvent mixture will generally be at a level between about 1% and about 20%, preferably about 2% to about 10%, even more preferably about 3% to about 5%, by weight with respect to the total weight of the composition. Suitable non-volatile solvents include, but are not limited to, benzyl benzoate, cetearyl alcohol, cetyl alcohol, diethyl phthalate, isopropyl myristate, dimethicone, caprylylmethicone and mixtures thereof.

Several other additional ingredients may be present in the compositions of the present invention. These include, but are not limited to, hydrophilic polymers selected from polyethylene glycols (PEG), polyvinylpyrrolidones (PVP), hydroxypropylmethylcellulose (HPMC) and poloxamers; UV stabilizers such as benzophenone-3; antioxidants such as tocopheryl acetate; preservatives such as phenoxyethanol, benzyl alcohol, methylparaben, propylparaben; pH adjusting agents such as lactic acid, citric acid, sodium citrate, succinic acid, phosphoric acid, sodium hydroxide, sodium carbonate; deodorants and antimicrobials, such as farnesol, zinc phenolsulfonate and ethylhexylglycerin; humectants such as tribehenin, glycerin; skin conditioning agents such as allantoin; cooling agents such as trimethyl isopropyl butanamide and menthol; hair treatment ingredients such as panthenol, pantethine, pantothine, panthenyl ethyl ether and their combinations; propellants such as propane, isopropane, butane and isobutene; salts in general, such as potassium acetate and sodium chloride and mixtures thereof; perfumes and dyes.

If present, these additional ingredients will preferably be present at a level of less than 10%, preferably less than 5% by weight of the total weight of the composition.

Preferably, in an embodiment according to the second object of the present invention, the pharmaceutical composition is for topical administration, more preferably in the form of lotion, cream, gel, stick, spray, ointment, paste, mousse; even more preferably it is in the form of a cream.

According to the first or second object of the present invention, the therapeutic treatment and/or prevention of skin dyschromia includes hypopigmentation and/or de-pigmentation disorders of the hypopigmentary skin, disorders of the melanic pigmentation of the skin, such as specific pathologies of post-trophic, neurotrophic, post-traumatic, post-infectious, post-surgical, autoimmune, genetic, metabolic, nutritional, endocrine, chemical, physical, dystrophic, degenerative or post-inflammatory origin.

Preferably, according to the first or second object of the present invention, the therapeutic treatment and/or prevention of skin dyschromia includes: vitiligo, bilateral vitiligo, acrofacial vitiligo, generalized vitiligo, focal vitiligo, segmental vitiligo, universal vitiligo, perinevic or Sutton's nevus vitiligo, leukoderma, skin dyschromia, piebaldism, pityriasis alba, pityriasis versicolor, idiopathic and post-inflammatory guttata hypomelanosis, acromic or depigmented nevi, progressive macular hypomelanosis, hypomelanosis caused by metabolic or nutritional or endocrine disorders, hypomelanosis caused by chemical, physical or pharmaceutical agents, infectious and post-infectious hypomelanosis, inflammatory edipomelanosis.

In an embodiment according to the first or second object of the invention relating to the peptide or composition for use in the therapeutic treatment and/or prevention of skin dyschromia, said peptide or said composition is in combination with a steroid-based compound or composition, or based on calcitriol.

In another embodiment according to the first or second object of the invention, relating to the peptide or composition for use in the therapeutic treatment and/or prevention of skin de-innervation diseases, said peptide or said composition is in combination with an antiviral compound or composition.

Preferably, the method of treatment and/or prevention of skin dyschromia comprises the oral, injectable or topical, simultaneous or sequential administration of a steroid-based or calcitriol-based compound or composition (activated vitamin D).

According to the first or second object of the present invention, the therapeutic treatment and/or prevention of skin de-innervation diseases includes skin de-innervation disorders of viral and/or inflammatory origin.

Preferably, according to the first or second object of the present invention, the therapeutic treatment and/or prevention of skin de-innervation diseases includes trigeminal trophic syndrome, post-herpes zoster itching, scalp dysesthesia, paresthetic notalgia, brachioradial itching, meralgia paresthetica, itching associated with keloids or burn scars, small fiber neuropathy in various systemic diseases, all of which are difficult to treat or lack effective treatment.

Preferably, the method of treatment and/or prevention of skin de-innervation diseases of viral origin comprises the oral, injectable or topical, simultaneous or sequential administration of an antiviral compound or composition.

Preferably, the method of treatment and/or prevention of skin dyschromia and/or skin de-innervation diseases does not require a photo-activation treatment, favoring adherence to the treatment by the patient.

The invention will be further described in the following examples, which do not limit the scope of the invention described in the claims.

## EXAMPLE 1

### In vitro activity

The in vitro ability of the NGF 1-14 peptide and the NGF whole protein to activate melanin production by melanocytes was compared.

According to already established protocols, primary cell cultures of normal human melanocytes were seeded in Petri dishes and cultured for one week, with and without the presence of the NGF 1-14 peptide at various concentrations, using the whole human NGF protein as a control. The melanin content was measured with the ELISA enzyme immunoassay. The maximum melanin concentration, measured as the difference from baseline, was experimentally determined from the plateau of the dose response curves of NGF and NGF 1-14. The EC50 represents the concentration of NGF 1-14 or NGF at which 50% of the maximum concentration of cellular melanin is observed. The experimental data are shown in Table 1.

TABLE 1

|  | NGF 1-14 | NGF |
| --- | --- | --- |
| EC50 | 1.2 $\mu$M | 60pM |

As reported in Table 1, the EC50 of NGF 1-14 is 1.2 $\mu$M, while NGF's EC50 is 60pM. These data show that NGF 1-14 is able to determine an increase in the melanin content per cell comparable to the whole protein NGF, even if at concentrations higher than 2-3 log.

On the other hand, proliferation and diffusion of melanocytes did not show significant differences.

## EXAMPLE 2

### 2a-In vivo activity - efficacy test

The in vivo capacity of skin re-pigmentation by the NGF 1-14 peptide and the whole NGF protein was compared.

The study was conducted on a mouse model of human vitiligo.

SCF transgenic mice of the C57BL6 line, treated for 10-12 weeks to induce vitiligo using CD8 + T cells specific for melanocytes, were shaved twice a week in the dorsal part affected by depigmentation, with an electric razor, in an area of about 6 cm x 6 cm. The animals were divided into two groups of equal numbers (8 animals per group). On a first group of animals, topical administration was carried out in two sites of the shaved area spaced from both sides of the midline, applying on the left a topical preparation containing 200 $\mu$g/mL of NGF peptide in a base cream vehicle, while on the right side only the base cream (control) was applied. The same procedure was repeated on the second group of animals, applying on the left a topical preparation containing 200 $\mu$g/mL of NGF 1-14 peptide in a base cream vehicle, while on the right side only the base cream (control) was applied.

The application was spread evenly twice a day for 8 weeks, in the central part of the shaved area, on a surface of about 2 cm x 2 cm. Starting from the second day and thereafter, the areas of interest were cleaned with a cloth before applying the preparations. The animals were kept away from possible sources of ultraviolet light irradiation.

Quantification of epidermal depigmentation was performed using a subjective scoring system based on a 0 to 5 point scale, where 5 represents the most severe degree of depigmentation, reported in the literature (Rebecca L. et al., Curr Protoc Immunol . (2019), 124(1) - February). A trained researcher blinded to the details of the experiment performed the evaluation at the treated site.

At the end of the study, 80% of the control animals treated with base cream showed a severe degree of depigmentation with a score greater than or equal to 4 both in the group of animals treated with NGF and in the group treated with NGF 1-14 for which the group of control was considered unique. Using the Assessment System described in the publication cited above, a clear effect on pigmentation was observed both in the group of animals treated with NGF 200 $\mu$g/mL in base cream and in the group treated with NGF 1-14 200 $\mu$g/mL in base cream.

In the first group of animals treated with NGF, 37.5% of the animals showed a degree of depigmentation less than or equal to 2, 37.5% showed a moderate degree equal to 3 and 25% a severe degree greater than or equal to 4.

In the second group of animals treated with NGF 1-14 at the same concentration 62.5% of the animals showed a degree of depigmentation less than or equal to 2, 25% showed a moderate degree equal to 3 only 12.5% a degree severe greater than or equal 4.

As shown by the above data, the complexion of the areas treated with the NGF 1-14 peptide showed a substantial reduction in depigmentation, compared with the complexion of the area treated with the vehicle alone.

In the comparison between peptide NGF 1-14 and NGF whole protein, the repigmentation was substantially comparable, but the percentage of animals with a degree of depigmentation less than or equal to 2 was found to be 62.5% in the group treated with NGF 1-14 compared 37.5% in the NGF-treated group.

The percentage of animals with a degree of depigmentation greater than or equal to 4 was also reduced by 50% in the group treated with NGF 1-14 compared to the group treated with whole protein.

Surprisingly with respect to in vitro activity, these data show that the NGF 1-14 peptide determines a more uniform and homogeneous repigmentation than the whole protein, at the same dose.

2b-Histological test

The in vivo ability of the NGF 1-14 peptide and the NGF whole protein to increase the density of nerve endings was compared.

After the treatment described in example 2a, the animals were sacrificed and skin samples (3 biopsies per point) were taken both from the area treated with NGF1-14 or with NGF, and from the untreated area, for each animal, for the purpose of histological examination.

To evaluate the density of sensory nerve fibers, antibodies directed against the neurofilament protein 200 (NF200; Sigma) specific for large diameter fibers, and antibodies directed against the transient receptor potential vanilloid 1 (TRPV1, Neuromics) were used to measure peripheral sensory fibers (small diameter).

The experimental data are shown in Table 2.

TABLE 2

| Parameters | Group 1 | | Group 2 | |
|---|---|---|---|---|
| | Base cream | NGF 1-14 200 $\mu$g/mL in base cream | Base cream | NGF 200 $\mu$g/mL in base cream |
| Nerve endings density (mm/mm$^3$) NF200 | 150 $\pm$ 72* | 380 $\pm$ 61 | 150 $\pm$ 72* | 350 $\pm$ 50 |
| Nerve termination density unit of measurement (mm/mm$^3$) TRPV1 | 120 $\pm$ 20* | 340 $\pm$ 20 | 150 $\pm$ 72* | 200 $\pm$ 36 |
| * the two groups were considered homogeneous with respect to the control | | | | |

The results of the histological analysis show a higher density of small and large diameter nerve fibers both in the group treated with NGF and in the group treated with NGF 1-14. As far as the small diameter fibers are concerned, there is a clear advantage of the treatment with NGF 1-14 compared to the treatment with the whole protein NGF.

Without wishing to bind to any interpretative theory, the Applicant believes that topical application on depigmented skin increases innervation in the treated area, exerting a neurotrophic action on de-pigmented or hypo-pigmented tissue. In turn, the increased innervation stimulates the cutaneous melanocytes and allows to obtain a repigmentation of the treated area.

**EXAMPLE 3**

In vivo activity - tolerability test

The in vivo intra-implant tolerability of the NGF 1-14 peptide was evaluated in comparison with the whole protein NGF.

Tyrode's isotonic solution containing the rh-NGF protein in concentrations from 5 to 20 $\mu$g/mL and the vehicle was administered intra-implantally in the dorsal region of Sprague-Dawley rats (10 animals for each concentration), in a volume of 0.05 mL using a very fine syringe.

Tyrode's isotonic solution containing the murine-NGF protein in concentrations from 5 to 20 $\mu$g/mL and the vehicle was administered intra-implantally in the dorsal region of Sprague-Dawley rats (10 animals for each concentration), in a volume of 0.05 mL using a very fine syringe.

Tyrode's isotonic solution containing the NGF 1-14 peptide in concentrations from 5 to 20 $\mu$g/mL and the vehicle was administered intra-implantally in the dorsal region of Sprague-Dawley rats (10 animals for each concentration), in a volume of 0.05 mL using a very fine syringe.

The solution was administered intra-implantally in the dorsal region of Sprague-Dawley rats (10 animals for each concentration), in a volume of 0.05 mL using a very fine syringe.

After the intra-implant injections, the Plantar thermal hyperalgesia test was conducted. In particular: after 1, 3, 5 and 24 h the thermal sensitivity was determined using the Hargreaves apparatus (HA).

Hypersensitivity to heat was assessed using the rat plantar test apparatus (Ugo Basile, Italy) following the method described by Hargreaves et al. (1988). The plantar test uses three perspex boxes (22x19x25 cm) placed on an elevated glass table. The rats were housed in each box, so that 3 rats could be tested simultaneously in a single apparatus and allowed to acclimate for at least 10 minutes. A mobile infrared heat source was applied to the plantar surface of the hind legs.

Paw retraction latency was defined as the time (in seconds) it took the rat to remove the hind leg from the heat source. The heat source was calibrated to an intensity of 15 IR and a cut-off of 50 sec was applied to prevent tissue damage. The test was performed at 1, 3, 5 and 24 hours after the injection. Each paw was tested twice per session (raw data represents the average of 2 measurements performed at an interval of approximately 5 minutes).

As shown in the graph shown in Figure 1, in the plantar test, the retraction latency of the paw of the animals treated with vehicle (saline) is unchanged for each time of the study (black circle - solid line). Rats treated with NGF 1-14 10$\mu$g/50 $\mu$L (white square), NGF 1-14 20$\mu$ g/50$\mu$ L (black circle - broken line) and rh-NGF 10$\mu$ g/50$\mu$ L mL (black triangle) showed hyperalgesic activity compared to the saline group at all time points of the study (P <0.001). In particular, rh-NGF 10 $\mu$g/50 $\mu$L showed a more pronounced significant hyperalgesic effect than NGF 1-14 at 10 and 20$\mu$ g/50$\mu$ L (* P <0.05 at 1-3-24h and ** P <0.01 at 5h).

The test results showed a high tolerability of NGF 1-14, even at the maximum dose of 20$\mu$g/50$\mu$L, much higher for each study time than the lowest dose of NGF.

Without wishing to bind to any interpretative theory, the Applicant believes that the unexpected tolerability of NGF 1-14 is due to the selectivity of action of the NGF 1-14 peptide towards the receptor tyrosine kinase TrKA with respect to the second receptor activated by NGF p75.

In conclusion, the NGF 1-14 peptide tested in vitro on primary cell cultures of normal human melanocytes results in a comparable increase in melanin content per cell compared to the whole protein even if at higher concentrations.

Surprisingly with respect to in vitro activity, the NGF 1-14 peptide tested in vivo on a mouse model of human vitiligo shows a more pronounced skin repigmentation, at the same dose, compared to the whole protein and a greater increase in the density of nerve endings, at the same dose.

Finally, the NGF 1-14 peptide administered intra-implant to Sprague-Dawley rats shows better tolerability than the NGF protein even at higher doses than those of the reference molecule. The result of the intra-implant test is very important, in light of the fact that the poor skin tolerability of NGF in intradermal tests is well known.

A further advantage of NGF1-14 over the whole protein is that, unlike the whole protein, it can be easily synthesized with conventional peptide synthesis approaches and stored under mild conditions and for prolonged periods, with a consequent significant reduction in production costs.

Therefore, the NGF 1-14 peptide such as peptides having sequence with at least 85%, preferably at least 90%, and preferably at least 95% identity with it and length up to 16 amino acids, are particularly useful in the treatment and/o in the prevention of skin dyschromia, such as vitiligo and skin de-innervation diseases.

Such oligo-peptide derivatives of NGF can therefore find application in the treatment and/or prevention of skin dyschromia, such as vitiligo, in a more effective and safe way than the cortisone derivatives currently used.

Furthermore, such oligo-peptide derivatives of NGF can find application in the treatment and/or prevention of skin de-innervation diseases, such as for example herpes, assisting antiviral therapy.

**Claims**

1. Peptide of 14 amino acids having the sequence ID No. 1

   or peptide of up to 16 amino acids having a sequence with at least 85%, preferably at least 90%, and preferably at least 95% identity with sequence ID No. 1,
   and/or salt thereof, for use in the therapeutic treatment and/or prevention of skin dyschromia and/or de-innervation diseases of the skin.

2. The peptide for use according to claim 1, having a sequence with at least 85%, preferably at least 90%, and preferably at least 95% identity with the sequence ID No. 1, wherein said peptide has a length of up to 15 amino acids, more preferably of 14 amino acids.

3. Pharmaceutical composition consisting of

a peptide of 14 amino acids having the sequence ID No. 1,
or a peptide of up to 16 amino acids having a sequence with at least 85%, preferably at least 90%, and preferably at least 95% identity with sequence ID No. 1,
and/or salt thereof,
and at least one pharmaceutically acceptable excipient,
for use in the therapeutic treatment and/or prevention of skin dyschromia and/or de-innervation diseases of the skin.

4. Pharmaceutical composition for use according to claim 3, said composition comprising from 10 to 10,000 μg/mL, preferably from 100 to 5,000 μg/mL, and more preferably from 200 to 4,000 μg/mL of said peptide.

5. Pharmaceutical composition for use according to claim 3 or 4 for topical administration, preferably in the form of lotion, cream, gel, stick, spray, ointment, paste, mousse.

6. Pharmaceutical composition for use according to any one of claims 3 to 5 wherein said at least one pharmaceutical acceptable excipient is selected from the group consisting of pharmaceutically acceptable vehicles, volatile and non-volatile solvents, water, surfactants, preservatives, absorbents, chelators, lubricants, moisturisers, water repellents, antioxidants, UV absorbers, anti-irritants, vitamins, trace metals, antimicrobial agents, perfumes, dyes and colored ingredients and/or structuring agents.

7. Peptide for use according to any one of claims 1 to 2, or composition for use according to any one of claims 3 to 6, wherein said skin dyschromia is selected from: vitiligo, bilateral vitiligo, acrofacial vitiligo, generalized vitiligo, focal vitiligo, segmental vitiligo, universal vitiligo, perinevic or Sutton's nevus vitiligo, leukoderma, skin dyschromia, piebaldism, pityriasis alba, pityriasis versicolor, idiopathic and post-inflammatory guttata hypomelanosis, acromic or depigmented nevi, progressive macular hypomelanosis, hypomelanosis caused by metabolic or nutritional or endocrine disorders, hypomelanosis caused by chemical, physical or pharmaceutical agents, infectious and post-infectious hypomelanosis, inflammatory edipomelanosis.

8. Peptide or composition for use according to claim 7 in combination with a steroid-based or calcitriol-based compound or composition.

9. Peptide for use according to any one of claims 1 to 2, or composition for use according to any one of claims 3 to 6, wherein said de-innervation disease of the skin is selected from: trigeminal trophic syndrome, post-herpes zoster itching, scalp dysesthesia, parestetic notalgia, brachioradial itching, meralgia paresthetica , itching associated with keloids or burn scars, small fiber neuropathy in various systemic diseases .

10. Peptide or composition for use according to claim 9 in combination with an antiviral compound or composition.

**Patentansprüche**

1. Peptid aus 14 Aminosäuren mit der Sequenz ID-Nr. 1

oder Peptid aus bis zu 16 Aminosäuren mit einer Sequenz mit mindestens 85 %, vorzugsweise mindestens 90 % und vorzugsweise mindestens 95 % Identität mit Sequenz ID-Nr. 1,
und/oder Salz davon zur Verwendung bei der therapeutischen Behandlung und/oder Vorbeugung von Hautdyschromie und/oder Deinnervations-Erkrankungen der Haut.

2. Peptid zur Verwendung nach Anspruch 1, das eine Sequenz mit mindestens 85 %, vorzugsweise mindestens 90 % und vorzugsweise mindestens 95 % Identität mit der Sequenz ID-Nr. 1 aufweist, wobei das Peptid eine Länge von bis zu 15 Aminosäuren, bevorzugter von 14 Aminosäuren aufweist.

3. Pharmazeutische Zusammensetzung, bestehend aus

einem Peptid aus 14 Aminosäuren mit der Sequenz ID-Nr. 1
oder einem Peptid aus bis zu 16 Aminosäuren mit einer Sequenz mit mindestens 85 %, vorzugsweise mindestens 90 % und vorzugsweise mindestens 95 % Identität mit der Sequenz ID-Nr. 1,
und/oder Salz davon,
und mindestens einem pharmazeutisch verträglichen Hilfsstoff
zur Verwendung bei der therapeutischen Behandlung und/oder Vorbeugung von Hautdyschromie und/oder Deinnervations-Erkrankungen der Haut.

4. Pharmazeutische Zusammensetzung zur Verwendung nach Anspruch 3, wobei die Zusammensetzung 10 bis 10.000 μg/ml, vorzugsweise 100 bis 5.000 μg/ml und bevorzugter 200 bis 4.000 μg/ml des Peptids umfasst.

5. Pharmazeutische Zusammensetzung zur Verwendung nach Anspruch 3 oder 4 zur topischen Verabreichung, vorzugsweise in Form von Lotion, Creme, Gel, Stift, Spray, Salbe, Paste, Mousse.

6. Pharmazeutische Zusammensetzung zur Verwendung nach einem der Ansprüche 3 bis 5, wobei der mindestens eine pharmazeutisch verträgliche Hilfsstoff aus der Gruppe ausgewählt ist, die aus pharmazeutisch verträglichen Trägern, flüchtigen und nichtflüchtigen Lösungsmitteln, Wasser, Tensiden, Konservierungsmitteln, Absorptionsmitteln, Chelatbildnern, Gleitmitteln, Feuchthaltemitteln, wasserabweisenden Mitteln, Antioxidantien, UV-Absorbern, Antireizmitteln, Vitaminen, Spurenmetallen, antimikrobiellen Mitteln, Parfüms, Farbstoffen und farbigen Inhaltsstoffen und/oder Strukturierungsmitteln besteht.

7. Peptid zur Verwendung nach einem der Ansprüche 1 bis 2 oder Zusammensetzung zur Verwendung nach einem der Ansprüche 3 bis 6, wobei die Hautdyschromie ausgewählt ist aus: Vitiligo, bilateraler Vitiligo, akrofazialer Vitiligo, generalisierter Vitiligo, fokaler Vitiligo, segmentaler Vitiligo, universeller Vitiligo, perinevischer oder Sutton-Nävus-Vitiligo, Leukodermie, Hautdyschromie, Piebaldismus, Pityriasis alba, Pityriasis versicolor, idiopathischer und postinflammatorischer Guttata-Hypomelanose, akromischer oder depigmentierter Nevi, progressiver makulärer Hypomelanose, Hypomelanose, die durch metabolische oder ernährungsbedingte oder endokrine Störungen verursacht wird, Hypomelanose, die durch chemische, physikalische oder pharmazeutische Mittel verursacht wird, infektiöser und postinfektiöser Hypomelanose, entzündlicher Edipomelanose.

8. Peptid oder Zusammensetzung zur Verwendung nach Anspruch 7 in Kombination mit einer Verbindung oder Zusammensetzung auf Steroidbasis oder Calcitriolbasis.

9. Peptid zur Verwendung nach einem der Ansprüche 1 bis 2 oder Zusammensetzung zur Verwendung nach einem der Ansprüche 3 bis 6, wobei die Deinnervations-Erkrankung der Haut ausgewählt ist aus: trigeminalem trophischem Syndrom, Post-Herpes-Zoster-Juckreiz, Kopfhautdysästhesie, parestetischer Notalgie, brachioradialem Juckreiz, Meralgia paresthetica, Juckreiz im Zusammenhang mit Keloiden oder Verbrennungsnarben, Kleinfaserneuropathie bei verschiedenen systemischen Erkrankungen.

10. Peptid oder Zusammensetzung zur Verwendung nach Anspruch 9 in Kombination mit einer antiviralen Verbindung oder Zusammensetzung.

**Revendications**

1. Peptide de 14 acides aminés ayant la séquence ID N° 1

   ou peptide de 16 acides aminés au maximum dont la séquence présente une identité d'au moins 85 %, de préférence d'au moins 90 %, et de préférence d'au moins 95 % avec la séquence ID N° 1,
   et/ou leur sel, pour une utilisation dans le traitement thérapeutique et/ou la prévention de la dyschromie cutanée et/ou des maladies de dénervation de la peau.

2. Peptide à utiliser selon la revendication 1, dont une séquence présente une identité d'au moins 85 %, de préférence d'au moins 90 %, et de préférence d'au moins 95 % avec la séquence ID N° 1, dans lequel ledit peptide a une longueur allant jusqu'à 15 acides aminés, plus préférentiellement de 14 acides aminés.

3. Composition pharmaceutique consistant en

un peptide de 14 acides aminés ayant la séquence ID N° 1,

ou un peptide de 16 acides aminés au maximum dont la séquence présente une identité d'au moins 85 %, de préférence d'au moins 90 %, et de préférence d'au moins 95 % avec la séquence ID N° 1,

et/ou leur sel,

et au moins un excipient pharmaceutiquement acceptable,

pour une utilisation dans le traitement thérapeutique et/ou la prévention de la dyschromie cutanée et/ou des maladies de dénervation de la peau.

4. Composition pharmaceutique à utiliser selon la revendication 3, ladite composition comprenant de 10 à 10 000 µg/ml, de préférence de 100 à 5 000 µg/ml, et plus préférentiellement de 200 à 4 000 µg/ml dudit peptide.

5. Composition pharmaceutique à utiliser selon la revendication 3 ou 4 pour administration topique, de préférence sous forme de lotion, crème, gel, bâtonnet, spray, pommade, pâte, mousse.

6. Composition pharmaceutique à utiliser selon l'une quelconque des revendications 3 à 5, dans laquelle ledit au moins un excipient pharmaceutiquement acceptable est choisi dans le groupe constitué de véhicules pharmaceutiquement acceptables, de solvants volatils et non volatils, d'eau, de surfactants, de conservateurs, d'absorbants, de chélateurs, de lubrifiants, d'hydratants, d'hydrofuges, d'antioxydants, d'absorbeurs d'UV, d'anti-irritants, de vitamines, de métaux traces, d'agents antimicrobiens, de parfums, de teintures et d'ingrédients colorés et/ou d'agents de structuration.

7. Peptide à utiliser selon l'une quelconque des revendications 1 à 2, ou composition à utiliser selon l'une quelconque des revendications 3 à 6, dans laquelle ladite dyschromie cutanée est choisie parmi : le vitiligo, le vitiligo bilatéral, le vitiligo acrofacial, le vitiligo généralisé, le vitiligo focal, le vitiligo segmentaire, le vitiligo universel, le vitiligo périnévique ou nævus de Sutton, la leucodermie, la dyschromie cutanée, le piébaldisme, le pityriasis alba, le pityriasis versicolor, l'hypomélanose guttate idiopathique et post-inflammatoire, les naevus acromiques ou dépigmentés, l'hypomélanose maculaire progressive, l'hypomélanose causée par des troubles métaboliques, nutritionnels ou endocriniens, l'hypomélanose causée par des agents chimiques, physiques ou pharmaceutiques, l'hypomélanose infectieuse et post-infectieuse, l'édipomélanose inflammatoire.

8. Peptide ou composition à utiliser selon la revendication 7 en combinaison avec un composé ou une composition à base de stéroïdes ou de calcitriol.

9. Peptide à utiliser selon l'une quelconque des revendications 1 à 2, ou composition à utiliser selon l'une quelconque des revendications 3 à 6, dans laquelle ladite maladie de dénervation de la peau est choisie parmi : le syndrome trophique du trijumeau, les démangeaisons post-zona, la dysesthésie du cuir chevelu, la notalgie paresthésique, les démangeaisons brachioradiales, la meralgie paresthésique, les démangeaisons associées aux chéloïdes ou aux cicatrices de brûlures, la neuropathie des petites fibres dans diverses maladies systémiques.

10. Peptide ou composition à utiliser selon la revendication 9 en combinaison avec un composé ou une composition antivirale.

**Fig. 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013065078 A2
- WO 2020075108 A1

**Non-patent literature cited in the description**

- **PROF. RITA LEVI-MONTALCINI**. Harvey Lect. Zoology Institute of the Washington University of St. Louis, 1966, vol. 60, 217
- **HEFTI F.** *J. Neurobiol.*, 1994, vol. 25, 1418
- **FRICKER J.** *Lancet*, 1997, vol. 349, 480
- **OLSON L. et al.** Parkinson's Disease and Dementia Section. *J. Neural Transm*, 1992, vol. 4, 79-95
- **PETTY BG et al.** *Annals of Neurology*, 1994, vol. 36, 244-246
- *J Biol Chem*, 1991, vol. 266, 21718-21722
- *J Invest Dermatol Symp Proc*, 1997, vol. 2, 31-36
- The trk family of receptors mediates nerve growth factor and neurotrophin-3 effects in melanocytes. *J. Clin. Invest.*, 1994, vol. 94, 1550-1562
- *Am. J. Clin. Dermatol.*, 2017, vol. 18, 733-744
- **ALTSCHUL et al.** *Mol. Biol.*, 1990, vol. 215, 403-410
- **ROWE et al.** Handbook of Pharmaceutical Excipients. *Pharmaceutical Press*, 2009
- **REBECCA L et al.** *Curr Protoc Immunol*, 2019, vol. 124 (1)